# DEMANDE DE BREVET EUROPEEN

(11) **EP 4 589 599 A1**
(43) Date de publication de la demande: **23.07.2025**
(21) Numéro de dépôt: 25150245.6
(22) Date de dépôt: 03.01.2025
(51) Int. Cl.: G16H 20/40, G16H 40/67, G16H 40/20

(54) **SYSTÈME DE SUIVI À DISTANCE D'UN APPAREIL DE FOURNITURE DE MONOXYDE D'AZOTE**

(30) Priorité: 19.01.2024 FR 2400542
(71) Demandeur: INOSYSTEMS, 92160 Antony (FR)
(72) Inventeur: BLANDIN, Yann, 92160 Antony (FR); DURAND, Stephanie, 92160 Antony (FR); SIDIBE, Yasmina, 92160 Antony (FR); MCODRUM, Anais, 92160 Antony (FR); MARCHAL, Frederic, 92160 Antony (FR)
(74) Mandataire: Air Liquide

(57) **Abrégé**

L'invention concerne un système (S) de suivi à distance d'un appareil de fourniture de NO (1) utilisé pour traiter des patients avec un gaz contenant du NO, comprenant des moyens de mémorisation de données de fonctionnement de l'appareil (1) recueillis pendant l'utilisation de l'appareil (1) au cours de traitements de patients successifs pendant une période de temps donnée (Dt). Les données de fonctionnement comprennent des déclenchements d'alarmes de différents types s'étant produits pendant la période de temps considérée. Un serveur distant (200) traite les données de fonctionnement mémorisées et en extrait des informations relatives aux traitements de patients ayant été réalisés et aux événements de fonctionnement survenus durant lesdits traitements de patients. Un afficheur graphique (300) affiche différentes informations relatives aux alarmes déclenchées.

## Description

L'invention concerne un système de suivi à distance d'au moins un appareil de fourniture de NO, de préférence de plusieurs appareils, utilisé pour traiter des patients avec un gaz contenant du NO, en particulier un mélange gazeux NO/N₂, c'est-à-dire un gaz à base de NO.

Le monoxyde d'azote est utilisé pour traiter les personnes, i.e. les patients, souffrant d'hypertension artérielle pulmonaire aiguë. Lorsqu'il est inhalé par le patient, le NO, appelé « NOi », « iNO » ou « NO inhalé », dilate les vaisseaux pulmonaires et augmente l'oxygénation en améliorant les échanges gazeux. Ces propriétés sont utilisées pour traiter différentes conditions médicales, comme l'Hypertension Artérielle Pulmonaire du Nouveau-né ou PPHN (pour *Persistent Pulmonary Hypertension of the Newborn*), le Syndrome de Détresse Respiratoire Aigüe ou SDRA observé principalement chez l'adulte ou les hypertensions pulmonaires (HP) en chirurgie cardiaque chez l'adulte ou l'enfant, comme décrit notamment par EP-A-560928, EP-A-1516639 et US-A-10,201,564.

Une installation de fourniture de gaz servant à la mise en oeuvre d'un traitement par NOi comprend habituellement une ou des bouteilles de NO/N₂ contenant un mélange gazeux NO/N₂ de composition donnée, un appareil de fourniture de NO alimenté en mélange NO/N₂ et un ventilateur médical fournissant un flux de gaz contenant de l'oxygène (i.e. >20 %vol. environ), tel de l'air, lesquels appareil de fourniture de NO et ventilateur médical alimentent un circuit patient raccordé fluidiquement à une interface respiratoire (i.e., sonde d'intubation trachéale, masque ou autre), lequel circuit comprend un capteur de débit et sert à véhiculer le flux gazeux. Il peut comprendre d'autres éléments, tel un humidificateur de gaz ou autre.

L'appareil de fourniture de NO et le ventilateur médical viennent se raccorder fluidiquement au circuit patient pour lui fournir le mélange gazeux NO/N₂ et le flux de gaz contenant de l'oxygène, respectivement.

Le mélange gazeux NO/N₂ alimentant l'appareil de fourniture de NO contient une faible quantité de NO gazeux (e.g. <1000 ppm vol.) dilué dans de l'azote (N₂). Il est ensuite injecté par l'appareil de fourniture de NO dans le flux gazeux contenant de l'oxygène, typiquement environ au moins 20%vol. d'oxygène (O₂), tel un mélange N₂/O₂ ou de l'air, voire de l'oxygène pur, provenant du ventilateur médical de manière à former un mélange gazeux final NO/N₂/O₂. Le mélange gazeux NO/N₂ subit donc une dilution dans le flux gazeux contenant de l'oxygène et le mélange gazeux final en résultant contient donc du NO, de l'azote et de l'oxygène, voire des impuretés inévitables.

Le mélange gazeux final NO/N₂/O₂ est véhiculé par le circuit patient et est ensuite administré par inhalation au patient au moyen de l'interface respiratoire, telle une sonde d'intubation trachéale, un masque respiratoire ou analogue, fournissant le NO gazeux aux voies respiratoires et/ou aux poumons du patient à traiter.

La concentration de NO finale dans mélange gazeux final (i.e. NO/N₂/O₂) administré au patient correspond à une posologie déterminée par un médecin ou analogue. En général, elle est comprise entre 1 et 80 ppm en vol. (ppmv), typiquement de l'ordre de 10 à 30 ppmv, en fonction de la population traitée, i.e. nouveau-nés, enfants, adolescents ou adultes, et/ou de la maladie à traiter.

L'appareil de fourniture de NO sert donc à réguler la quantité de mélange NO/N₂ injectée dans le flux gazeux à base d'oxygène provenant du ventilateur et à assurer un suivi du traitement, notamment des quantités de NO fournies, des valeurs de consigne de NO réglées, des seuils d'alarme réglés (e.g. NO, NO₂...), des déclenchements d'alarme éventuels ou d'autres réglages ou évènements s'étant déroulés pendant le traitement du ou des patients. Autrement dit, l'appareil de fourniture de NO permet d'assurer une fourniture de NO et un suivi, i.e. monitorage, de cette fourniture, et de déclencher des alarmes sonores et visuelles, en cas de détection de problèmes liés.

Lorsqu'un appareil de fourniture de NO est utilisé en milieu hospitalier, on constate de nombreux déclenchements d'alarmes ayant des criticités différentes, qui requièrent toutes une action de la part du personnel soignant. Ainsi, chaque alarme déclenchée doit être constatée par le personnel soignant qui doit en comprendre l'origine et, le cas échéant, mettre en oeuvre une ou des mesures correctives appropriées.

Pour ce faire, actuellement, le personnel soignant doit consigner par écrit, par exemple sur un registre, cahier ou analogue, la liste de toutes les alarmes qui se sont déclenchées ainsi que d'autres informations liées, comme le/les appareils concerné(s), le/les patients concernés, la date et l'heure des déclenchements...., afin de pouvoir visualiser après coup quelles alarmes se sont déclenchées sur la période de temps considérée et surtout de comprendre les raisons de ces déclenchements d'alarmes de manière à pouvoir y apporter les mesures correctives adaptées.

Si les alarmes sont indispensables pour la sécurité du patient, elles sont également génératrices de stress pour le personnel soignant et chronophage du fait notamment des consignations à opérer dans le registre.

De plus, ces consignations par écrit faites par le personnel soignant sont sources d'erreurs et d'omission conduisant à des erreurs d'interprétation, voire à la mise en place d'actions correctives non-adaptées pouvant impacter l'efficacité des traitements futurs, donc la sécurité des patients.

Ceci est encore plus vrai lorsque plusieurs appareils de fourniture de NO sont utilisés au sein d'un même établissement hospitalier.

Par ailleurs, US2021268221 qui enseigne un dispositif pour générer du NO à partir d'un gaz réactif (e.g. N₂ et O₂), au moyen d'électrodes agencées dans une ou des chambres à plasma. Un contrôleur sert à réguler quantité de NO produite. Un cartouche de ventilateur remplaçable sert à héberger des capteurs surveillant des flux et pouvant comprendre une mémoire servant à enregistrer différentes informations, comme les informations de configuration, les numéros de lot ou de série, les logs patient ou d'alarmes, les historiques, etc... Il comprend un moniteur d'affichage des courbes des patients et des alarmes pouvant être relié à distance à un système de visualisation.

En outre, le manuel d'utilisation de l'appareil de fourniture de NO par inhalation appelé INOmax DS_{IR} Plus, 2014-07, permet d'enregistrer les alarmes déclenchées, au sein d'une mémoire de l'appareil. L'historique des alarmes est consultable sur l'appareil lui-même.

Ces appareils ne permettent pas d'opérer un suivi à distance des alarmes, en particulier lorsque plusieurs appareils de NO doivent être suivis simultanément, tel un parc d'appareils utilisés au sein d'un même établissement hospitalier.

Un problème est donc de pouvoir éviter tout ou partie de ces problèmes, en particulier ceux-ci liés à la nécessité de tenir un registre des alarmes pour chaque appareil de fourniture de NO, notamment afin d'améliorer l'efficacité des traitements par iNO et la sécurité des patients. On comprend que ce problème est d'autant plus important que le nombre d'appareils de fourniture de NO est élevé, sachant que dans certains hôpitaux ou analogues, une flotte de plusieurs dizaines d'appareils peut être utilisée pour traiter des patients.

Autrement dit, il est primordial de pouvoir assurer un suivi efficace, i.e. un monitorage, au fil du temps d'un ou plusieurs appareils de fourniture de NO utilisés pour traiter des patients en milieu hospitalier, en particulier d'une flotte d'appareils de fourniture de NO utilisés au sein d'un même établissement hospitalier, lesquels appareils sont susceptibles de déclencher chacun des alarmes durant leur fonctionnement, c'est-à-dire lorsqu'ils sont utilisés pour traiter des patients.

Une solution de l'invention concerne un système de suivi à distance, i.e. de monitorage, d'au moins un appareil de fourniture de NO utilisé pour traiter des patients avec un gaz contenant du NO, en particulier mélange gazeux NO/N₂, comprenant des moyens de mémorisation pour mémoriser des données de fonctionnement de l'appareil considéré recueillis pendant l'utilisation de l'appareil considéré au cours de traitements de patients successifs ayant eu lieu pendant une période de temps donnée (Dt), chaque appareil de fourniture de NO étant alimenté en gaz contenant du NO, typiquement un mélange gazeux NO/N₂, au cours desdits traitements de patients, lesdites données de fonctionnement comprenant des déclenchements d'alarmes de différents types s'étant produits pendant ladite période de temps donnée (Dt).

Ledit système de suivi à distance comprend :
- au moins un serveur distant configuré pour traiter les données de fonctionnement mémorisées par les moyens de mémorisation de l'appareil de fourniture de NO et en extraire des informations relatives aux traitements de patients ayant été réalisés et aux évènements de fonctionnement survenus durant lesdits traitements de patients, et
- un afficheur graphique configuré pour afficher au moins une partie desdites informations, les informations affichées par l'afficheur graphique comprenant, pour au moins un intervalle de temps (It) considéré de la période de temps donnée (Dt) :
   ▪ au moins une partie des alarmes les plus fréquemment déclenchées, i.e. les plus fréquentes,
   ▪ une occurrence ou un nombre de déclenchements d'au moins une partie des alarmes les plus fréquemment déclenchées,
   ▪ une proportion de déclenchement d'au moins une partie des alarmes les plus fréquemment déclenchées,
   ▪ un temps moyen de résolution d'au moins une partie des alarmes les plus fréquemment déclenchées,
   ▪ un nombre moyen d'alarmes déclenchées par traitement et/ou
   ▪ un temps moyen de résolution d'au moins une partie des alarmes les plus fréquemment déclenchées.

De plus, le système de suivi à distance comprend en outre des moyens de sélection de la durée de l'intervalle de temps (It) configurés pour permettre, à un utilisateur, de choisir une date de début de période et une date de fin de période, ou une date de début de période et une durée.

Selon le mode de réalisation considéré, le système de suivi à distance de l'invention peut comprendre l'une ou plusieurs des caractéristiques suivantes :
- l'afficheur graphique est configuré pour afficher plusieurs informations différentes choisies parmi les informations susmentionnées.
- les informations affichées par l'afficheur graphique comprennent les 3 à 20 alarmes les plus fréquemment déclenchées, i.e. les plus fréquentes, sur l'intervalle de temps (It) considéré, de préférence les 5 à 15 alarmes les plus fréquemment déclenchées, par exemple des 10 alarmes les plus fréquemment déclenchées.
- les informations affichées par l'afficheur graphique comprennent des alarmes dites « critiques » ou alarmes critiques choisies parmi les alarmes les plus fréquemment déclenchées, i.e. les plus fréquentes, sur l'intervalle de temps (It) considéré, de préférence les 3 à 7 alarmes critiques. Les alarmes critiques correspondent aux alarmes les plus fréquemment déclenchées présentant les plus fortes occurrences, c'est-à-dire les plus nombreuses ou s'étant déclenchées le plus souvent sur l'intervalle de temps (It) considéré.
- les informations affichées par l'afficheur graphique comprennent les occurrences ou nombres de déclenchements des alarmes dites « critiques » ou alarmes critiques.
- les informations affichées par l'afficheur graphique comprennent la fréquence ou proportion (%) de déclenchements des alarmes dites « critiques » ou alarmes critiques.
- l'afficheur graphique est du type à affichage en couleurs.
- l'afficheur graphique est en outre configuré pour afficher l'intervalle de temps (It) considéré, par exemple une date de début et une date de fin.
- l'afficheur graphique est en outre configuré pour afficher, pour l'intervalle de temps (It) considéré : un nombre total de traitements, un nombre de traitements courts, de préférence de moins de 6 heures, et/ou un nombre de traitements longs, de préférence d'au moins 6 heures.
- l'afficheur graphique est en outre configuré pour afficher pour l'intervalle de temps (It) considéré, 3 à 6 alarmes critiques correspondant aux 3 à 6 alarmes les plus fréquemment déclenchées présentant les occurrences les plus élevées, par exemple 5 alarmes critiques.
- l'afficheur graphique est en outre configuré pour afficher les alarmes les plus fréquemment déclenchées et/ou les alarmes critiques en fonction de leur occurrence, par exemple une liste commençant avec l'alarme présentant la plus forte occurrence et se terminant avec celle présentant la plus faible occurrence.
- l'afficheur graphique est en outre configuré pour afficher les alarmes les plus fréquemment déclenchées et/ou les alarmes critiques dans une ou plusieurs fenêtres d'affichage affichées sur l'afficheur graphique.
- les moyens de sélection de la durée de l'intervalle de temps (It) sont configurés pour permettre de sélection un intervalle de temps (It), i.e. une durée, de plusieurs semaines, de préférence de plusieurs mois, par exemple de 2 à 12 mois, en particulier de 3 à 8 mois, par exemple une durée de 1 année.
- l'appareil de fourniture de NO est alimenté en NO gazeux provenant d'un ou plusieurs récipients de NO contenant ledit gaz à base de NO, typiquement le mélange gazeux NO/N₂.
- l'appareil de fourniture de NO est alimenté en un mélange gazeux NO/N₂ provenant d'un ou plusieurs récipient de NO, typiquement d'une ou plusieurs bouteilles de NO, contenant le mélange gazeux NO/N₂.
- l'appareil de fourniture de NO comprend des moyens de télécommunication configurés pour transmettre les données de fonctionnement de l'appareil vers ledit au moins un serveur distant.
- les moyens de télécommunication sont configurés pour fonctionner en utilisant un réseau mobile, en particulier un réseau de type 4G, 5G ou analogue, ou autre.
- les moyens de télécommunication comprennent des moyens d'émission, typiquement ils comprennent (au moins) une antenne émettrice.
- les moyens de télécommunication comprennent des moyens d'émission configurés pour émettre des données sous forme cryptée et/ou analogique.
- ledit au moins un serveur distant comprend un serveur de type « cloud » (« nuage »), par exemple le « cloud » AWS (Amazon Web Service).
- ledit au moins un serveur distant comprend des moyens de réception pour recevoir les données de fonctionnement transmises par l'appareil de fourniture de NO.
- les types d'alarmes (i.e. les types d'alarmes identifiés) sont choisis parmi :
   ▪ une alarme de concentration de NO « non conforme » correspondant à une valeur de concentration analysée très différente de la valeur de concentration réglée, par exemple avec un écart d'au moins 30%, de préférence d'au moins 40%, de préférence encore d'au moins 50% de la dose cible.
   ▪ une alarme de concentration de NO trop basse,
   ▪ une alarme de concentration de NO trop élevée,
   ▪ une alarme de FiO₂ trop basse,
   ▪ une alarme de FiO₂ trop élevée,
   ▪ une alarme de défaut de batterie, c'est-à-dire de dysfonctionnement de la batterie (e.g. vide, en panne....),
   ▪ une alarme de défaut de la ligne d'injection de NO,
   ▪ une alarme de défaut de la ligne de mesure de débit, c'est-à-dire un dysfonctionnement du capteur de débit,
   ▪ une alarme de défaut de flux gazeux provenant du ventilateur (i.e. air ou mélange O₂/N₂), par exemple d'absence de flux gazeux ou, à l'inverse, de flux excessif,
   ▪ une alarme de défaut de connexion patient, par exemple de patient débranché ou branché/connecté mais sans administration de flux de NO,
   ▪ une alarme de défaut de piège à eau,
   ▪ une alarme de défaut de ligne d'analyse, i.e. de dysfonctionnement de la ligne d'analyse ou d'échantillonnage et
   ▪ une alarme de défaut de source de gaz (NO, O₂), par exemple de non-raccordement ou de détection de bouteille de gaz vide (i.e. bouteille de NO et/ou bouteille d'O₂), typiquement une alarme d'absence totale de bouteille de NO pleine raccordée à l'appareil de fourniture de NO, i.e. alarme de bouteille de NO vide.
- de préférence, les types d'alarmes comprennent en outre :
   ▪ une alarme d'activation du système de ventilation manuelle (i.e. via un ballon de ventilation manuelle (BAVU) venant se raccorder au appareil de fourniture de NO),
   ▪ une alarme d'activation du mode d'administration d'urgence,
   ▪ une alarme de fonctionnement sur batterie, i.e. quand le cordon d'alimentation électrique est débranché ou en cas de défaut d'alimentation secteur (i.e. 110V/220V), et/ou
   ▪ une alarme de batterie vide ou quasiment vide, par exemple ayant une autonomie inférieure à 10% de l'autonomie maximale (i.e. batterie pleinement chargée).
- il comprend en outre des moyens de traitement informatique configurés pour recevoir et traiter au moins une partie des informations traitées fournies par ledit au moins un serveur distant.
- les moyens de traitement informatique sont en outre configurés pour contrôler l'affichage des informations affichées par l'afficheur graphique.
- les moyens de traitement informatique mettent en oeuvre au moins un algorithme configuré pour identifier chaque traitement, en extraire les évènements recherchés/suivis, et traiter ces évènements pour en déterminer les alarmes liées.
- l'afficheur graphique est configuré pour afficher au moins une partie des informations relatives à chacun desdits appareils de fourniture de NO.
- il configuré pour suivre à distance plusieurs appareils de fourniture de NO.
- il configuré pour suivre à distance de 2 à 50 appareils de fourniture de NO utilisés au sein d'un même établissement hospitalier. En effet, dans les établissements hospitaliers les plus importants, il peut être nécessaire de suivre simultanément un parc d'appareils, typiquement plusieurs dizaines d'appareils de fourniture de NO.
- il configuré pour suivre à distance au moins 5 appareils de fourniture de NO utilisés au sein d'un même établissement hospitalier, de préférence au moins 10 appareils, de préférence encore au moins 15 à 20 appareils.
- il comprend des moyens de sélection configurés pour permettre à un utilisateur de choisir ou sélectionner l'appareil dont il souhaite connaitre les informations, i.e. les alarmes, c'est-à-dire un appareil donné parmi une pluralité d'appareils, i.e. un parc d'appareils de fourniture de NO, utilisés au sein d'un établissement hospitalier.
- les moyens de sélection comprennent une ou des touches d'un clavier d'ordinateur ou autre, un écran tactile, une souris et/ou analogue.
- le ou chaque appareil de fourniture de NO fait partie d'une installation d'administration de gaz comprenant en outre un ventilateur médical pour fournir un gaz contenant de l'oxygène et un circuit patient alimenté par l'appareil de fourniture de NO en gaz contenant du NO et par le ventilateur médical en gaz contenant de l'oxygène.

Selon le mode de réalisation considéré, le ou chaque appareil ou dispositif de fourniture d'un gaz contenant du NO faisant partie du système de suivi à distance de l'invention, peut comprendre l'une ou plusieurs des caractéristiques suivantes :
- il comprend au moins un passage interne, typiquement un circuit de gaz comprenant plusieurs passages de gaz, par exemple un ou plusieurs conduits de gaz, passages, tuyaux ou analogues, pour acheminer le flux de gaz contenant du NO et des moyens à valve agencés sur ledit au moins un passage interne, par exemple une ou des valves ou vannes, en particulier une ou des électrovannes ou une ou des vannes tout ou rien.
- il comprend des moyens de pilotage à microprocesseur(s), à savoir un contrôleur ou analogue, comprenant un ou plusieurs microprocesseurs.
- les moyens de pilotage à microprocesseur(s) coopèrent (au moins) avec les moyens à valve pour contrôler le débit de gaz dans au moins une partie dudit passage interne ou circuit de gaz interne.
- les moyens de pilotage sont configurés pour commander les moyens de télécommunication, en particulier pour contrôler les transmissions des données de fonctionnement de l'appareil vers ledit au moins un serveur distant.
- le ou les microprocesseurs sont agencés sur une carte électronique.
- les moyens de mémorisation comprennent un dispositif de mémorisation informatique, telle une mémoire informatique, par exemple une mémoire flash.
- la mémoire informatique est agencée sur la ou une carte électronique.
- il comprend des moyens d'alimentation électrique, telle une liaison électrique au secteur (110/220V).
- il comprend une interface graphique utilisateur (IGU), de préférence l'IGU comprend un écran d'affichage et/ou des touches de sélection, telles des touches virtuelles s'affichant sur l'écran d'affichage, i.e. un écran à dalle tactile.

De plus, la ou chaque installation d'administration de gaz à un patient, i.e. d'un gaz contenant du NO, incluant le ou chaque appareil ou dispositif de fourniture d'un gaz contenant du NO comprend :
- au moins une source de gaz contenant du NO gazeux, en particulier un mélange gazeux NO/N₂, de préférence une ou des récipients de gaz, telles une ou des bouteilles de gaz sous pression,
- un appareil de fourniture de gaz selon l'invention, en particulier comme décrit ci-avant, alimenté(s) en gaz contenant du NO par ladite au moins une source de gaz, telle mélange gazeux NO/N₂,
- un ventilateur médical pour fournir un gaz contenant de l'oxygène, tel de l'air ou un mélange gazeux O₂/N₂, et
- une ligne d'alimentation alimentée par le appareil de fourniture de gaz en gaz contenant du NO, telle mélange gazeux NO/N₂, et par le ventilateur médical gaz contenant de l'oxygène, tel de l'air ou le mélange O₂/N₂.

Selon le mode de réalisation considéré, la ou chaque installation d'administration de gaz peut comprendre l'une ou plusieurs des caractéristiques suivantes :
- le (chaque) ventilateur médical, c'est-à-dire un appareil d'assistance ventilatoire, est en communication fluidique avec la ligne d'alimentation pour alimenter ladite ligne d'alimentation avec un gaz respiratoire contenant au moins environ 20% vol. d'oxygène, de préférence au moins environ 21% vol. d'oxygène, en particulier de l'air ou un mélange N₂/O₂.
- le (chaque) ventilateur médical est un appareil d'assistance respiratoire fournissant le gaz à pression constante ou, alternativement, le ventilateur médical est un ventilateur de type HFO (*High Frequency Oscillations*) délivrant le gaz par oscillations à haute fréquence.
- la ligne d'alimentation en gaz est alimentée en mélange NO/N₂ par le (chaque) appareil de fourniture de gaz et en un gaz respiratoire contenant au moins environ 20% vol. d'oxygène, de préférence au moins environ 21% vol. d'oxygène, de préférence de l'air ou un mélange N₂/O₂, par le ventilateur médical.
- la ligne d'alimentation en gaz est alimentée en mélange NO/N₂ par l'appareil de fourniture de gaz et en un gaz respiratoire contenant de l'oxygène, de préférence de l'air ou un mélange N₂/O₂, par le ventilateur médical de manière à y former un mélange gazeux final à administrer au patient contenant du NO, de l'azote et de l'oxygène, voire d'autres composés comme de la vapeur d'eau et/ou des impuretés, comme l'argon ou des espèces NO₂ formées par oxydation d'une partie du NO.
- le mélange gazeux final à administrer au patient contient de l'azote, de l'oxygène et du NO en une proportion correspondant à une dose de NO préfixée, c'est-à-dire une posologie.
- le mélange gazeux final à administrer au patient contient au moins 20%vol. d'oxygène, de 150 à 1000 ppmv de NO et de l'azote, et éventuellement des impuretés inévitables et/ou de la vapeur d'eau.
- la (ou les) source de gaz contient un mélange gazeux NO/N₂ contenant moins de 2 000 ppmv de NO, le reste étant de l'azote, de préférence moins de 1 000 ppmv de NO, le reste étant de l'azote.
- préférentiellement, la (ou les) source de gaz thérapeutique contient un mélange NO/N₂ contenant de 250 à 900 ppmv de NO, le reste étant de l'azote, par exemple de l'ordre de 800 ppmv de NO, le reste étant de l'azote.
- elle comprend en outre un humidificateur de gaz agencé sur la ligne d'alimentation en gaz, de préférence en aval du site où le dispositif de fourniture de gaz thérapeutique est raccordé fluidiquement à ladite ligne d'alimentation en gaz de manière à l'alimenter en gaz thérapeutique.
- elle comprend en outre une ligne de récupération des gaz expirés par le patient.
- la ligne d'alimentation en gaz et la ligne de récupération des gaz expirés sont raccordées à une pièce de raccordement, de préférence une pièce en Y, et définissent ou forment tout ou partie d'un circuit patient.
- la ligne d'alimentation forme une branche inspiratoire du circuit patient.
- la ligne de récupération des gaz expirés forme une branche expiratoire du circuit patient.
- l'appareil de fourniture de NO comprend en outre une ligne d'analyse de gaz reliée fluidiquement à la ligne d'alimentation en gaz, i.e. la branche inspiratoire.
- la ligne d'alimentation en gaz, i.e. la branche inspiratoire, comprend un capteur de débit agencé entre le ventilateur et le site d'injection du gaz contenant le NO provenant de l'appareil de fourniture de NO.
- le capteur de débit est relié aux moyens de pilotage de l'appareil de fourniture de NO.
- le capteur de débit mesure le débit de gaz contenant de l'oxygène (i.e. air ou mélange N₂/O₂) délivré par le ventilateur médical et circulant dans la ligne d'alimentation en gaz, i.e. la branche inspiratoire.
- le capteur de débit retourne des valeurs ou des signaux de débit de gaz.
- les moyens de pilotage sont configurés pour contrôler les moyens à valve pour délivrer le flux gazeux contenant du NO (e.g. mélange NO/N₂) à un débit donné permettant d'obtenir une dose de NO dans la ligne d'alimentation en gaz correspondant à la dose souhaitée, i.e. la posologie fixée par le personnel soignant.
- la ligne d'alimentation en gaz alimente une interface respiratoire, par exemple un masque respiratoire, une sonde d'intubation trachéale ou analogue.
- la ligne d'alimentation en gaz, i.e. la branche inspiratoire, est raccordée fluidiquement à un port de sortie du ventilateur médical de manière à récupérer et acheminer le gaz délivré par le ventilateur médical.
- la ligne de récupération des gaz expirés, i.e. la branche expiratoire, est raccordée fluidiquement à un port d'entrée du ventilateur médical de manière à acheminer jusqu'au ventilateur médical tout ou partie des gaz expirés par le patient.
- au moins une source de gaz thérapeutique comprend un ou plusieurs récipients de gaz, en particulier une ou des bouteilles de gaz sous pression.
- le (ou les) récipient de gaz est équipé d'un robinet de distribution de gaz avec ou sans détenteur intégré (RDI).
- le robinet de distribution de gaz est en alliage de cuivre, tel du laiton, et/ou est équipé d'un capotage de protection agencé autour du robinet de distribution de gaz, par exemple en matériau polymère (i.e. plastique), en métal ou leurs combinaisons.
- le (ou les) récipient de fluide est (sont) une bouteille de gaz sous pression contenant, lorsqu'il est plein, un mélange gazeux, en particulier NO/N₂, à une pression d'au moins 135 à 200 bar abs, voire d'au moins 250 à 300 bar abs.
- le récipient de fluide a une forme générale cylindrique, en particulier d'ogive.

D'une façon générale, dans le cadre de l'invention :
- « ppmv » signifie partie par million en volume,
- « %vol. » » signifie pourcentage en volume.
- « NO » désigne le monoxyde d'azote.
- « NO₂ » désigne le dioxyde d'azote.
- « N₂ » désigne l'azote.
- « O₂ » désigne l'oxygène.
- les termes « concentration », « dose » et « teneur » sont considérés comme équivalents.
- les termes « appareil » et « dispositif » sont considérés comme équivalents.
- les termes « fourniture » et « délivrance » sont considérés comme équivalents.
- les termes « moyen de/à/pour » sont considérés comme totalement équivalents et substituables par les termes « dispositif de/à/pour », par exemple les termes « moyens de pilotage » peuvent être remplacés par « dispositif de pilotage », les termes « moyens à valves » peuvent être remplacés par « dispositif à valves », les « moyens de mémorisation» peuvent être remplacés par « dispositif de mémorisation» ...

L'invention va maintenant être mieux comprise grâce à la description détaillée suivante, faite à titre illustratif mais non limitatif, en référence aux figures annexées parmi lesquelles :
Fig. 1 schématise un mode de réalisation d'un système de suivi à distance selon l'invention d'un appareil de fourniture de NO, en particulier un appareil de fourniture de NO faisant partie d'une installation d'administration de gaz.
Fig. 2 schématise un mode de réalisation d'une installation d'administration de gaz comprenant un appareil de fourniture de NO pouvant être suivi à distance par un système de suivi à distance selon l'invention, tel celui de Fig. 1.
Fig. 3 schématise un exemple des affichages opérés par un système de suivi à distance selon l'invention, tel celui de Fig. 1.

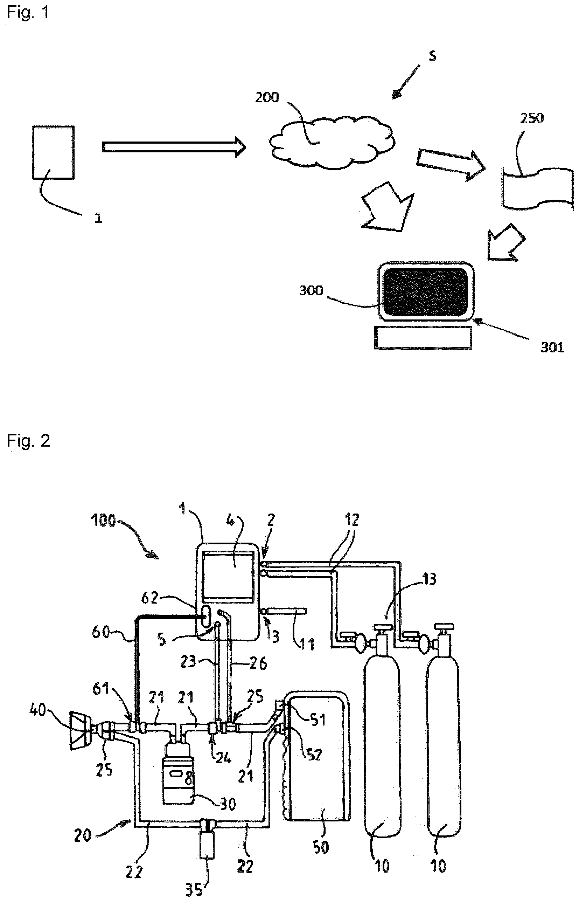

Fig. 2 schématise un mode de réalisation d'une installation d'administration de gaz 100 comprenant un appareil de fourniture de NO 1, c'est-à-dire d'un mélange gazeux à base de monoxyde d'azote, pouvant être suivi à distance par un système S de suivi à distance selon l'invention, tel celui schématisé sur Fig. 1, comme expliqué ci-après.

Plus précisément, l'installation 100 comprend ici deux bouteilles de gaz sous pression 10 contenant chacune un mélange gazeux NO/N₂, à savoir ici un mélange gazeux NO/N₂ contenant typiquement entre 100 et 1500 ppmv de NO (reste N₂), par exemple 450 ou 800 ppmv de NO (reste N₂), ou toute autre concentration adéquate, qui alimentent en mélange NO/N₂, le dispositif ou appareil 1 de fourniture, i.e. de délivrance, de NO permettant de suivre et contrôler la fourniture du mélange gazeux NO/N₂.

Les bouteilles de gaz 10 sont reliées fluidiquement à l'appareil 1 de fourniture de NO, via des lignes d'amenée de gaz 12, tel que des tuyaux ou conduits flexibles ou analogues, qui peuvent être équipées de dispositifs de régulation et/ou de suivi de la pression de gaz, tels que détendeur de gaz 13, manomètres... Les lignes d'amenée de gaz 12 sont reliées à une ou plusieurs entrées de gaz 2 du dispositif de délivrance de NO 1 qui alimentent un passage de gaz interne servant à acheminer le gaz au sein de l'appareil de fourniture de NO 1, c'est-à-dire dans le boitier ou la carcasse externe de l'appareil 1.

L'appareil de fourniture de NO 1 comprend aussi une entrée d'oxygène 3 reliée fluidiquement, via une ligne d'amenée d'oxygène 11, tel un tuyau flexible ou analogue, à une source d'oxygène (non montrée), par exemple une bouteille d'oxygène sous pression ou un réseau hospitalier, c'est-à-dire une canalisation d'alimentation en oxygène agencée dans un bâtiment hospitalier.

L'installation d'administration de gaz 100 comprend par ailleurs un ventilateur médical 50, c'est-à-dire un appareil d'assistance respiratoire, qui fournit un flux de gaz respiratoire à base d'oxygène, c'est-à-dire contenant au moins 20%vol. d'oxygène environ, préférentiellement au moins 21%vol. d'oxygène environ, tel de l'air ou un mélange oxygène/azote (N₂/O₂).

Le ventilateur médical 50 et l'appareil de fourniture de NO 1 de l'installation d'administration de gaz 100 sont en communication fluidique avec une ligne d'alimentation en gaz ou branche inspiratoire 21 d'un circuit patient 20. La ligne d'alimentation en gaz ou branche inspiratoire 21 sert à acheminer le flux gazeux vers le patient (non montré), en particulier le mélange gazeux final à administrer au patient qui est formé par mélange du flux à base d'oxygène (e.g. air ou mélange NO/N₂) provenant du ventilateur médical 50 et du flux à base de NO, i.e. le mélange gazeux NO/N₂, délivré par l'appareil de fourniture de NO 1.

Plus précisément, l'appareil de fourniture de NO 1 délivre ou injecte le mélange NO/N₂, par exemple à 450 ou 800 ppmv de NO, dans la ligne d'alimentation en gaz 21, via un conduit ou ligne d'injection 23, reliant fluidiquement le circuit de gaz interne (non visible sur Fig. 2) de l'appareil de fourniture de NO 1 à la ligne d'alimentation en gaz 21.

Le flux de NO/N₂ amené par la ligne d'injection 23 se mélange, en un site d'injection 24, au flux de gaz à base d'oxygène (> 20% d'O₂) délivré par le ventilateur médical 50 et amené par la portion amont de la branche inspiratoire 21 du circuit patient 20 de sorte d'obtenir le mélange final à administrer au patient, lequel contient essentiellement du NO à la posologie désirée, de l'azote (N₂) et de l'oxygène (O₂), et éventuellement des impuretés inévitables (e.g. argon, CO₂, NO₂, ....), c'est-à-dire un mélange gazeux final NO/N₂/O₂.

De préférence, la branche inspiratoire 21 comprend en outre un humidificateur de gaz 30 agencé en aval du site d'injection 24 où se fait l'injection de NO dans la branche inspiratoire 21. Il permet d'humidifier le flux de gaz final avant son administration au patient.

Le mélange gazeux NO/N₂/O₂ est ensuite administré par inhalation au patient à traiter au moyen d'une interface respiratoire 40, telle une sonde d'intubation trachéale, un masque respiratoire ou analogue, permettent de délivrance le gaz final (i.e. NO/N₂/O₂) dans les poumons du patient.

Une ligne de récupération des gaz expirés par le patient forme une branche expiratoire 22 du circuit patient 20. Elle est reliée fluidiquement à la ligne d'alimentation en gaz ou branche inspiratoire 21 via une pièce de raccordement 25, telle une pièce en Y.

La branche inspiratoire 21 est raccordée, en amont, fluidiquement à un port de sortie 51 du ventilateur médical 50, tel un connecteur, raccord ou analogue, de manière à récupérer et acheminer le gaz à base d'oxygène, typiquement de l'air ou mélange N₂/O₂, venant du ventilateur médical 50, alors que la branche expiratoire 22 véhiculant les gaz expirés est raccordée fluidiquement à un port d'entrée 52 du ventilateur médical 50, tel un connecteur, raccord ou analogue, de manière à retourner au ventilateur médical 50 tout ou partie du débit des gaz expirés par le patient. La branche expiratoire 22 peut comprendre un ou plusieurs composants optionnels, par exemple un dispositif d'élimination du CO₂ 35, i.e. un piège à CO₂, tel un bac à chaud ou autre, permettant d'éliminer le CO₂ présent dans les gaz expirés par le patient, un filtre ou autre.

Un capteur de débit 25, par exemple du type à fil chaud, à différentiel de pression ou massique, est agencé sur la ligne d'alimentation en gaz 21, entre le ventilateur 50 et l'humidificateur 30, et est relié à l'appareil de délivrance de NO 1, via une (des) ligne de mesure de débit 26.

Le capteur de débit 25 sert à mesurer le débit de gaz délivré par le ventilateur 50 circulant dans la branche inspiratoire 21, en amont du site d'injection 24 où se raccorde la ligne d'injection 23 et se fait le mélange gazeux NO/N₂/O₂. Ceci permet de réguler plus efficacement la délivrance du flux de NO (i.e. N₂/O₂) par l'appareil de fourniture de NO 1 puisque les mesures de débit opérées par le capteur de débit 25 sont retournées, via la ligne de mesure de débit 26, à des moyens de pilotage (non montrés) embarqués dans l'appareil de délivrance de NO 1. Typiquement, les moyens de pilotage comprennent une carte électronique comprenant un (ou plusieurs) microprocesseur(s), typiquement un (ou des) (micro)contrôleur, mettant en oeuvre un ou des algorithmes.

Plus précisément, l'appareil de fourniture de NO 1 comprend un boitier rigide, par exemple en polymère, comprenant un circuit de gaz interne (non visible), tel un (des) conduit de gaz ou analogue, servant à acheminer le flux de gaz à base de NO, i.e. le mélange NO/N₂, provenant des bouteilles de mélange de NO/N₂ 12, et/ou le flux d'oxygène provenant de la source d'oxygène 11.

Le circuit de gaz interne de l'appareil de fourniture de NO 1 relie fluidiquement l'entrée (ou les entrées) de gaz 21 de l'appareil 1 à la ligne d'injection 23 afin de convoyer le flux de gaz à base de NO. Des moyens à valve (non montrés), i.e. un (ou des) dispositif à valve(s), par exemple une pluralité d'électrovannes agencées en parallèle, préférentiellement une ou des (électro)vannes proportionnelles, sont agencés sur le circuit de gaz interne de l'appareil 1 pour contrôler le flux gazeux qui y circule en direction de la ligne d'injection 23 et du site d'injection 24.

Les moyens à valve de l'appareil de fourniture de NO 1 sont eux-mêmes commandés par les moyens de pilotage, i.e. un (ou des) dispositif de pilotage, aussi appelé (micro)contrôleur, agencés dans le boitier de l'appareil de fourniture de NO 1.

Les moyens de pilotage permettent notamment d'ajuster ou contrôler le débit de gaz à base de NO en pilotant les moyens à valve, typiquement d'ouvrir ou fermer cette ou ces vannes, pour obtenir un débit de gaz à base de NO déterminé, lequel a été calculé par les moyens de pilotage à partir d'une valeur de teneur en NO réglée et/ou fixée par l'utilisateur, typiquement une posologie souhaitée, et en fonction du débit de gaz, e.g. air ou N₂/O₂, délivré par le ventilateur 50, lequel est mesuré par le capteur de débit 25 agencé sur la branche inspiratoire 21 et relié au dispositif de fourniture de NO 1, en particulier aux moyens de pilotage, par la ligne de mesure de débit 26.

Le circuit de gaz interne de l'appareil de fourniture de NO 1 peut aussi comprendre un (ou des) débitmètre agencé en amont et/ou en aval des moyens à valve, pour déterminer le débit de gaz à base de NO circulant dans l'appareil de fourniture de NO 1, notamment pour s'assurer qu'il est conforme au débit souhaité. Le débitmètre peut être du type à différentiel de pression, à fil chaud ou autre. Il coopère avec les moyens de pilotage pour leur fournir des mesures de débit du flux de NO/N₂. Ces mesures de débit sont traitées par les moyens de pilotage afin d'assurer une délivrance efficace de NO en fonction notamment du débit de gaz à base d'O₂ fourni par le ventilateur médical 50.

De préférence, le circuit de gaz interne de l'appareil de fourniture de NO 1 peut aussi comprendre un régulateur de pression, tel un détendeur de gaz ou analogue, afin d'ajuster, e.g. réduire, la pression du gaz à base de NO provenant des bouteilles de gaz 10.

Habituellement, l'appareil de fourniture de NO 1 comprend par ailleurs une interface graphique utilisateur ou IGU comprenant un afficheur graphique 4, de préférence un écran tactile, c'est-à-dire à dalle tactile, servant à afficher différentes informations ou données, icones, courbes, alarmes..., ainsi que des touches de sélection virtuelles et/ou des pavés ou fenêtres, servant notamment à opérer des choix, des sélections ou encore à entrer des informations, telles des valeurs désirées (e.g. débit, dosage de NO...), ou toute autre information ou donnée utile au personnel soignant. De préférence, l'affichage est en couleurs mais il peut se faire aussi en noir et blanc.

Les moyens de pilotage de l'appareil de fourniture de NO 1 comprennent au moins une carte de commande électronique et au moins une unité de contrôle à microprocesseur, typiquement un microcontrôleur ou analogue. Les moyens de pilotage permettent en outre d'opérer des calculs et/ou de contrôler ou commander tous les éléments électromécaniques de l'appareil 1. Plus précisément, la carte de commande intègre préférentiellement l'unité de contrôle et est configurée pour commander et par ailleurs analyser et/ou traiter les signaux provenant des différents composants, tels que les capteurs...

L'alimentation électrique de l'appareil de fourniture de NO 1, en particulier des composants nécessitant du courant électrique pour fonctionner, tels les moyens de pilotage, l'afficheur graphique 4..., est assurée classiquement par une source de courant électrique et/ou des moyens d'alimentation électrique (non montrés), par exemple une liaison au courant du secteur (110/220V) de type cordon électrique et prise de raccordement, et/ou une (ou des) batterie d'alimentation électrique, de préférence rechargeable, et/ou un transformateur de courant. L'alimentation électrique du ventilateur médical 50 est assurée de façon analogue, notamment par une liaison au courant du secteur ou une batterie interne.

Enfin, l'installation 100 comprend aussi une ligne de prélèvement de gaz 60 qui relie fluidiquement la branche inspiratoire 21 à l'appareil de fourniture de NO 1. Elle vient se raccorder fluidiquement (en 61) à la ligne d'alimentation en gaz 21, en aval du site d'injection 24, à savoir ici entre l'humidificateur 30 et la pièce de jonction 25, i.e. pièce en Y, typiquement à proximité immédiate de la pièce de jonction 25, et par ailleurs à un port d'entrée 62 du dispositif de fourniture de NO 1, par exemple un port 62 porté par un connecteur, raccord ou analogue, permettant le raccordement de la ligne de prélèvement de gaz 60, tel un tuyau flexible ou analogue.

La ligne de prélèvement de gaz 60 permet de prélever des échantillons de gaz dans la branche inspiratoire 21 du circuit patient 20 et de les convoyer jusqu'au dispositif de fourniture de NO 1 où ils sont analysés dans un analyseur de gaz interne (non montré), c'est-à-dire au sein d'une ligne de calibration comprenant au moins un capteur relié électriquement aux moyens de pilotage, afin de vérifier leur conformité. Typiquement, on prévoit une ligne de calibration comprenant des capteurs de NO₂, de NO et d'O₂, telles des cellules électrochimiques ou analogues, qui doivent être calibrés périodiquement, par exemple toutes les semaines.

En effet, il convient de vérifier que la composition du gaz final est conforme à celle du mélange gazeux NO/N₂/O₂ souhaité devant être administré au patient, notamment pour s'assurer qu'il ne contient pas de quantité excessive d'espèces NO₂ toxiques, que sa teneur en oxygène n'est pas hypoxique (i.e. <20%vol), et que sa teneur en NO correspond à la posologie souhaitée, i.e. dose de NO à administrer au patient qui est habituellement choisie par le personnel soignant, i.e. médecin ou analogue.

Les moyens de pilotage de l'appareil 1 sont configurés pour récupérer et traiter, i.e. analyser, les signaux provenant des différents capteurs de l'analyseur de gaz et d'agir en réponse à ces signaux, notamment pour déclencher une alarme en cas de détection d'une concentration non-conforme, par exemple excessive en NO₂, insuffisante en O₂ (i.e. hypoxique) ou différente de la posologie en NO désirée.

Une telle installation d'administration de gaz 100 peut être utilisée pour administrer par inhalation du monoxyde d'azote (NOi), i.e. le mélange final obtenu NO/O₂/N₂, aux personnes, i.e. patients, souffrant d'hypertension artérielle pulmonaire aiguë, notamment pour opérer une dilatation de leurs vaisseaux pulmonaires et une augmentation de leur oxygénation en améliorant les échanges gazeux pulmonaires, en particulier pour traiter l'Hypertension Artérielle Pulmonaire du Nouveau-né ou PPHN, le Syndrome de Détresse Respiratoire Aigüe ou SDRA observé principalement chez l'adulte, ou les hypertensions pulmonaires (HP) en chirurgie cardiaque chez l'adulte ou l'enfant.

Au sein d'un établissement hospitalier, i.e. hôpital ou analogue, il arrive régulièrement que plusieurs appareils de fourniture 1 de NO soient utilisés au sein d'un service donné et/ou dans différents services, comme aux urgences, en réanimation, en cardiologie..., c'est-à-dire un parc ou une flotte d'appareils de fourniture 1 de NO. Il est courant de retrouver au moins 5 à 10 appareils au sein d'un même établissement hospitalier, voire plus, par exemple jusqu'à 50 appareils pour les établissements les plus importants. Ces appareils peuvent être utilisés (au moins certains d'entre eux) simultanément ou alternativement.

Toutefois, chacun de ces appareils de fourniture 1 de NO permet d'assurer une fourniture de NO et un suivi, i.e. monitorage, de cette fourniture au fil du temps, notamment lors de traitements de patient successifs, et de déclencher des alarmes sonores et visuelles, en cas de détection de problèmes survenus lors de ces traitements de patient successifs.

Afin d'éviter au personnel soignant d'avoir à consigner par écrit tous les déclenchements d'alarmes et informations liées pour chacun des appareils, on propose selon l'invention un système de suivi à distance S permettant de suivre un ou plusieurs appareil(s) 1 de fourniture de NO, en particulier d'un d'appareils 1 mis en oeuvre dans un établissement hospitalier, tel un hôpital ou analogue, pour soigner des patients avec du iNO. Lorsqu'ils sont utilisés, chaque appareil est alimenté en NO comme expliqué ci-avant.

Ainsi, Fig. 1 schématise un mode de réalisation d'un tel système de suivi à distance selon l'invention d'un ou plusieurs appareil(s) 1 de fourniture de NO, en particulier de l'appareil 1 de fourniture de NO faisant partie de l'installation d'administration de gaz 100 de Fig. 2 détaillée ci-avant.

D'une façon générale, dans le système S de suivi à distance de Fig. 1, chaque appareil de fourniture de NO 1 est doté de moyens de mémorisation, à savoir une mémoire informatique ou analogue, configurée pour mémoriser des données de fonctionnement de chaque appareil 1 considéré recueillis pendant son utilisation ou son fonctionnement, c'est-à-dire au cours des traitements de patients successifs ayant eu lieu pendant une période de temps donnée Dt, y compris sur des durées longues, par exemple de plusieurs semaines ou plusieurs mois, typiquement sur une période de 2 à 18 mois, préférentiellement une période de 6 à 12 mois.

Les données de fonctionnement enregistrées comprennent en particulier les déclenchements d'alarmes de différents types ayant été déclenchés pendant la période de temps considérée Dt par chaque appareil 1 mais aussi toutes les informations liées à ces déclenchements, à savoir la date et l'heure de chaque déclenchement d'alarme, la référence de l'appareil 1 concerné, une identification du traitement, le site hospitalier concerné ou autres.

De plus, le système S comprend aussi un (ou des) serveur distant 200, c'est-à-dire un serveur informatique, par exemple un serveur de type Cloud (nuage) ou autre, configuré pour traiter les données de fonctionnement mémorisées par chaque appareil de fourniture de NO 1 et en extraire des informations traitées relatives aux traitements de patients ayant été réalisés et aux évènements de fonctionnement survenus durant lesdits traitements de patients, en particulier ceux ayant conduit aux différents déclenchements d'alarmes.

Afin de pouvoir transmettre les données de fonctionnement de chaque appareil 1, chaque appareil fourniture de NO 1 comprend des moyens de communication configurés pour transmettre au serveur informatique distant 200 qui comprend, quant à lui, des moyens de réception pour recevoir les données de fonctionnement transmises par l'appareil de fourniture de NO 1.

Plus précisément, le système 1 comprend aussi des moyens de traitement informatique 250, pouvant être intégré dans le serveur 200, qui sont configurés pour recevoir et traiter les informations qui leur sont fournies par le serveur distant 200 et pour ensuite contrôler l'affichage des informations affichées par un afficheur graphique 300.

Les moyens de traitement informatique 250 comprennent un ou plusieurs (micro)processeurs mettant en oeuvre des algorithmes.

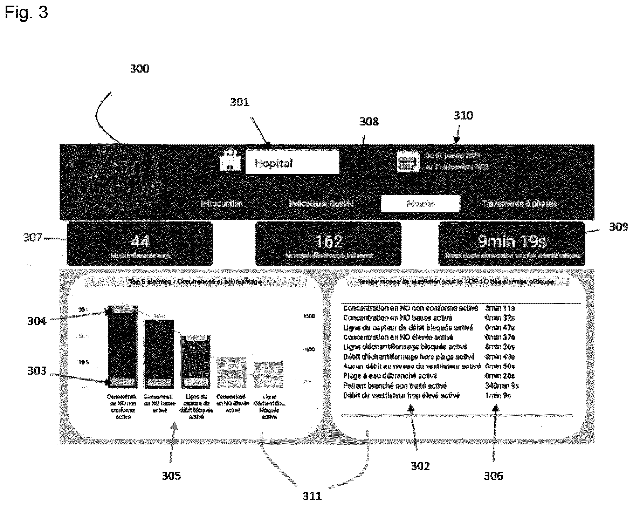

Autrement dit, le serveur distant 200 coopère avec un afficheur graphique 300, par exemple celui d'un ordinateur 301, comme illustré en Fig. 1 et Fig. 3, d'une tablette numérique ou analogue, servant à afficher tout ou partie des informations traitées par le serveur distant 200, en particulier par les moyens de traitement informatique 250, par exemple les informations recueillies et mémorisées correspondant à un intervalle de temps (It) déterminé, par exemple 1 à 3 mois (ou une autre durée) de la période de temps donnée (Dt), par exemple une période Dt de 12 mois.

De préférence, le système S comprend des moyens de sélection permettant de sélectionner la durée de l'intervalle de temps (It), par exemple une durée de plusieurs semaines ou de plusieurs mois, par exemple de 2 à 12 mois, en particulier de 3 à 8 mois. Ceci peut se faire en choisissant une date de début de période et une date de fin de période, ou une date de début de période et une durée, par exemple 3 mois.

De plus, on prévoit aussi des moyens de sélection permettant à un utilisateur de sélectionner un appareil particulier dont il souhaite connaitre les informations, i.e. les alarmes ou autres, au sein du parc d'appareils suivis. Les moyens de sélection comprennent par exemple une ou des touches ou analogue.

En particulier, selon l'invention, les informations affichées par l'afficheur graphique 300 comprennent de préférence :
- les alarmes les plus fréquemment déclenchées 302, c'est-à-dire une liste d'alarmes fréquentes, typiquement des 3 à 15 alarmes fréquentes, par exemple les 10 alarmes les plus fréquemment déclenchées, et préférentiellement les 5 alarmes plus critiques 305 ou les plus fréquemment déclenchées, dites alarmes critiques (ou top alarmes), parmi ces alarmes les plus fréquemment déclenchées 302,
- une occurrence de déclenchement 304 (i.e. nombre) d'au moins une partie des alarmes les plus fréquemment déclenchées 302, par exemple le nombre d'alarmes critiques 305 déclenchées,
- la proportion (%) 303 d'au moins une partie des alarmes les plus fréquemment déclenchées, par exemple de celle des alarmes critiques 305.
- un temps de résolution 309 moyen des alarmes les plus fréquemment déclenchées 302, c'est-à-dire de la durée s'écoulant entre un déclenchement d'alarme et l'acquittement de cette alarme par l'utilisateur, par exemple par appui sur une touche d'acquittement, par résolution du problème ou autre.
- un nombre moyen d'alarmes déclenchées par traitement 308,
- un temps moyen (ou durée moyenne) de résolution 306 de chacune des alarmes les plus fréquemment déclenchées 302 et/ou
- un temps moyen (ou durée moyenne) de résolution 309 des alarmes les plus fréquemment déclenchées 302.

De préférence, l'afficheur graphique 300 est configuré pour afficher plusieurs des informations de cette liste, en particulier les alarmes déclenchées.

Avantageusement, les différents types d'alarmes pouvant être affichées sont par exemple :
- une alarme de concentration de NO (trop) basse, e.g. inférieure à la dose désirée,
- une alarme de concentration de NO (trop) élevée, e.g. supérieure à la dose désirée,
- une alarme de concentration de NO dite « non conforme », c'est-à-dire très éloignée de la dose désirée, par exemple quand l'écart de concentration est d'au moins 30%, de préférence d'au moins 40%, de préférence encore d'au moins 50% de l'attendu). Cet écart peut être préfixé et/ou mémorisé.
- une alarme de FiO₂ (trop) basse, e.g. inférieure à la FiO₂ désirée,
- une alarme de FiO₂ (trop) élevée, e.g. supérieure à la FiO₂ désirée,
- une alarme de dysfonctionnement de batterie (vide, en panne....),
- une alarme de défaut de la ligne d'injection de NO,
- une alarme de défaut de la ligne de mesure de débit, c'est-à-dire un dysfonctionnement du capteur de débit,
- une alarme de défaut de flux gazeux provenant du ventilateur, par exemple un flux insuffisant ou interrompu, ou trop élevé,
- une alarme de patient connecté sans administration de flux à base de NO,
- une alarme de patient non-branché, c'est-à-dire ne recevant pas de flux à base de NO,
- une alarme de piège à eau dysfonctionnant, par exemple débranché ou saturée d'humidité,
- une alarme de ligne d'analyse dysfonctionnant, i.e. de la ligne d'échantillonnage, et
- une alarme de détection d'une bouteille de gaz (NO, O₂) vide, c'est-à-dire dont le gaz a été consommé.

Bien entendu, d'autres types d'alarmes peuvent aussi être aussi prises en compte et affichées sur l'afficheur graphique 300.

De préférence, l'afficheur graphique 300 est en outre configuré pour afficher, pour l'intervalle de temps (It) considéré 310, un nombre total de traitements, c'est-à-dire de patients ayant été traités par iNO et/ou, parmi ceux-ci, le nombre de traitements courts (TC), c'est-à-dire de traitements ayant eu une durée inférieure ou égale à une durée maximale donnée, par exemple une durée maximale de 6h (ou une autre durée donnée), et/ou le nombre de traitements longs (TL) 307, c'est-à-dire ayant eu une durée supérieure à la durée maximale donnée, par exemple de plus de 6h.

Fig. 3 donne un exemple d'affichages opérées sur l'afficheur graphique 300 d'un système selon l'invention S, tel celui de Fig. 1.

On y voit que sont affichées, pendant une période de temps considéré pouvant être fixée par l'utilisateur, typiquement par sélection d'une date de début et d'une date de fin de période 310, par exemple ici entre le 01 janvier et le 31 décembre 2023 :
- l'hôpital concerné 301, par exemple le nom et l'adresse de l'hôpital,
- les 5 alarmes dites critiques 302 (appelées « Top 5 Alarmes ») parmi les 10 alarmes les plus fréquemment déclenchées 305,
- la proportion (%) 303 de chacune de ces alarmes dites critiques 302.
- le nombre ou occurrences 304 de chacune de ces alarmes dites critiques 302,
- la liste des 10 alarmes les plus fréquemment déclenchées et leur durée de résolution moyenne 306,
- le nombre de traitement longs 307, par exemple d'au moins 6h,
- le nombre moyen d'alarmes déclenchées par traitement 308, et
- la durée moyenne ou temps moyen de résolution 309 des 5 alarmes critiques 305.

Comme on le voit ici, parmi les 5 alarmes dites critiques 305, l'alarme la plus fréquente est celle relative à une concentration de NO dite « non conforme » signifiant que la dose de NO mesurée est très éloignée de la dose réglée, c'est-à-dire que l'écart est par exemple d'au moins 50%. Ceci se rencontre quand l'équipe de soin débranche la ligne d'analyse en cours de traitement; par exemple pendant des soins de nébulisation/aérosolthérapie. Enregistrer cette alarme et sa récurrence permet de détecter ce dysfonctionnement d'utilisation de l'appareillage et de le corriger en indiquant au personnel soignant comment mieux utiliser les filtres spéciaux dits « pour nébulisation/aérosolthérapie », alors que la moins fréquente est celle liée à un dysfonctionnement de la ligne d'échantillonnage (dite « bloquée »).

Dans le mode de réalisation présenté en Fig. 3, la liste des alarmes les plus fréquemment déclenchées 305 et celle des 5 alarmes dites critiques 305 sont affichées dans des fenêtres 311 d'affichage différentes, à savoir ici juxtaposées, préférentiellement des fenêtres 311 d'affichage de grande taille afin de faciliter la lecture.

D'une façon générale, le système S de l'invention permet de suivre à distance plusieurs appareils de fourniture de NO 1 utilisés au sein d'un établissement hospitalier et l'afficheur graphique 300 permet d'afficher des informations relatives à chacun des appareils de fourniture de NO 1 suivis à distance et utilisés pour mettre en oeuvre des traitements de personnes, i.e. des patients, par NO inhalé (iNO), notamment souffrant d'hypertension artérielle pulmonaire aiguë, telle l'Hypertension Artérielle Pulmonaire du Nouveau-né (PPHN), le Syndrome de Détresse Respiratoire Aigüe (SDRA) ou les hypertensions pulmonaires (HP) en chirurgie cardiaque.

## Revendications

1. Système (S) de suivi à distance d'au moins un appareil de fourniture de NO (1) utilisé pour traiter des patients avec un gaz contenant du NO, et comprenant des moyens de mémorisation pour mémoriser des données de fonctionnement de l'appareil (1) recueillis pendant l'utilisation de l'appareil (1) au cours de traitements de patients successifs ayant eu lieu pendant une période de temps donnée (Dt), le ou chaque appareil de fourniture de NO (1) étant alimenté en gaz contenant du NO au cours desdits traitements de patients, lesdites données de fonctionnement comprenant des déclenchements d'alarmes de différents types s'étant produits pendant ladite période de temps donnée (Dt),
ledit système (S) comprenant :
- au moins un serveur distant (200) configuré pour traiter les données de fonctionnement mémorisées par les moyens de mémorisation du ou de chaque appareil de fourniture de NO (1) et en extraire des informations relatives aux traitements de patients ayant été réalisés et aux évènements de fonctionnement survenus durant lesdits traitements de patients, et
- un afficheur graphique (300) configuré pour afficher au moins une partie desdites informations, les informations affichées par l'afficheur graphique (300) comprenant, pour au moins un intervalle de temps (It) considéré de la période de temps donnée (Dt) :
▪ au moins une partie des alarmes les plus fréquemment déclenchées (302, 305),
▪ une occurrence ou un nombre de déclenchement (304) d'au moins une partie des alarmes les plus fréquemment déclenchées,
▪ une proportion de déclenchement (303) d'au moins une partie des alarmes les plus fréquemment déclenchées,
▪ un temps moyen de résolution (306) d'au moins une partie des alarmes les plus fréquemment déclenchées,
▪ un nombre moyen (308) d'alarmes déclenchées par traitement et/ou
▪ un temps moyen de résolution (309) d'au moins une partie des alarmes les plus fréquemment déclenchées,
**caractérisé en ce qu'**il comprend en outre des moyens de sélection de la durée de l'intervalle de temps (It) configurés pour permettre de choisir une date de début de période et une date de fin de période, ou une date de début de période et une durée.

2. Système selon la revendication 1, **caractérisé en ce que** les informations affichées par l'afficheur graphique (300) comprennent les 3 à 15 alarmes les plus fréquemment déclenchées sur l'intervalle de temps (It) considéré.

3. Système selon la revendication 1, **caractérisé en ce que** l'afficheur graphique (300) est en outre configuré pour afficher en outre :
- l'intervalle de temps (It) considéré (310), et/ou
- pour l'intervalle de temps (It) considéré :
▪ un nombre total de traitements, un nombre de traitements courts et/ou un nombre de traitements longs (307), et/ou
▪ des alarmes critiques (305) choisies parmi les alarmes les plus fréquemment déclenchées (302), de préférence de 3 à 7 alarmes critiques (305).

4. Système selon l'une des revendications 1 à 3, **caractérisé en ce que** la durée de l'intervalle de temps (It) est de plusieurs semaines, de préférence de plusieurs mois.

5. Système selon la revendication 1, **caractérisé en ce que** :
- le ou chaque appareil de fourniture de NO (1) comprend des moyens de communication configurés pour transmettre les données de fonctionnement du ou de chaque appareil (1) audit au moins un serveur distant (200), et
- ledit au moins un serveur distant (200) comprend des moyens de réception pour recevoir les données de fonctionnement transmises par l'appareil de fourniture de NO (1).

6. Système selon la revendication 1, **caractérisé en ce que** les types d'alarmes sont choisis parmi :
- une alarme de concentration de NO « non conforme »,
- une alarme de concentration de NO trop basse,
- une alarme de concentration de NO trop élevée,
- une alarme de FiO₂ trop basse,
- une alarme de FiO₂ trop élevée,
- une alarme de défaut de batterie,
- une alarme de défaut de la ligne d'injection de NO,
- une alarme de défaut de la ligne de mesure de débit,
- une alarme de défaut de flux gazeux provenant du ventilateur,
- une alarme de défaut de connexion patient,
- une alarme de défaut de piège à eau,
- une alarme de défaut de ligne d'analyse et
- une alarme de défaut de source de gaz (NO, O₂).

7. Système selon la revendication 1, **caractérisé en ce qu'**il comprend en outre des moyens de traitement informatique (250) configurés pour recevoir et traiter au moins une partie des informations fournies par ledit au moins un serveur distant (200), et contrôler l'affichage des informations affichées par l'afficheur graphique (300).

8. Système selon la revendication 7, **caractérisé en ce que** les moyens de traitement informatique (250) mettent en oeuvre au moins un algorithme.

9. Système selon la revendication 1, **caractérisé en ce qu'**il est configuré pour suivre à distance plusieurs appareils de fourniture de NO (1).

10. Système selon l'une des revendications 1 ou 9, **caractérisé en ce qu'**il est configuré pour suivre à distance de préférence de 2 à 50 appareils de fourniture de NO (1), de préférence au moins 5 appareils.

11. Système selon la revendication 1, **caractérisé en ce qu'**il comprend des moyens de sélection configurés pour permettre à un utilisateur de choisir ou sélectionner un appareil de fourniture de NO donné parmi une pluralité d'appareils de fourniture de NO utilisés au sein de l'établissement hospitalier.

12. Système selon l'une des revendications 1 à 4, **caractérisé en ce que** la durée de l'intervalle de temps (It) est de 2 à 12 mois, en particulier de 3 à 8 mois.

13. Système selon l'une des revendications 1 à 4 ou 12, **caractérisé en ce que** l'afficheur graphique (300) est en outre configuré pour afficher l'intervalle de temps (It) considéré, de préférence une date de début et une date de fin.

14. Système selon la revendication 3, **caractérisé en ce que** l'afficheur graphique (300) est configuré pour afficher une fréquence ou proportion (%) de déclenchements des alarmes critiques.

15. Système selon la revendication 1, **caractérisé en ce que** le ou chaque appareil de fourniture de NO (1) fait partie d'une installation d'administration de gaz (100) comprenant en outre un ventilateur médical (50) pour fournir un gaz contenant de l'oxygène et un circuit patient (20) alimenté par l'appareil de fourniture de NO (1) en gaz contenant du NO et par le ventilateur médical (50) en gaz contenant de l'oxygène.
